# EUROPEAN PATENT SPECIFICATION

(11) **EP 2 586 360 B1**
(45) Date of publication and mention of the grant of the patent: **04.10.2023**
(21) Application number: 11798006.0
(22) Date of filing: 13.06.2011
(51) Int. Cl.: A61B 3/10, A61B 3/06

(54) **LENS ASSESSMENT DEVICE, CONTROL PROGRAM, AND CONTROL METHOD OF LENS ASSESSMENT DEVICE**
LINSENTESTVORRICHTUNG, STEUERPROGRAMM DAFÜR UND STEUERVERFAHREN FÜR DIE LINSENTESTVORRICHTUNG
DISPOSITIF D'ÉVALUATION DE LENTILLE, PROGRAMME DE COMMANDE, ET PROCÉDÉ DE COMMANDE DE DISPOSITIF D'ÉVALUATION DE LENTILLE

(30) Priority: 23.06.2010 JP 2010142490
(43) Date of publication of application: 01.05.2013
(73) Proprietor: Ichikawa, Kazuo, Nagoya-shi, Aichi 465-0012 (JP); Shinshu University, Matsumoto-shi, Nagano 390-8621 (JP); Kowa Company, Ltd., Nagoya-shi, Aichi 460-8625 (JP)
(72) Inventor: TANAKA, Kiyoshi, Nagano City Nagano 380-8553 (JP); TANAKA, Yoshiki, Nagano City Nagano 380-8553 (JP); ICHIKAWA, Kazuo, Nagoya-shi, Aichi 465-0012 (JP)
(74) Representative: Gulde & Partner
(86) International application number: PCT/JP2011/063488
(87) International publication number: WO 2011/162120

(56) References cited:
- JP-A- 11 032 991
- JP-A- 2004 537 331
- JP-A- 2005 253 583
- JP-A- 2005 278 942
- JP-A- 2008 237 492
- US-B1- 6 203 157
- M.-CH LALANNE ET AL: "COMMUNICATION DE LA SFO Pseudophakie et aptitude du personnel navigant Pseudophakia and flying personnel aptitude", J. FR. OPHTALMOL, vol. 22, no. 3, 1 January 1999 (1999-01-01), pages 353-358, XP055262730,
- P R KINNEAR ET AL: "New Farnsworth-Munsell 100 hue test norms of normal observers for each year of age 5-22 and for age decades 30-70", BRITISH JOURNAL OF OPHTHALMOLOGY, vol. 86, no. 12, 1 December 2002 (2002-12-01), pages 1408-1411, XP055263295, GB ISSN: 0007-1161, DOI: 10.1136/bjo.86.12.1408
- SCHWIEGERLING J: "Recent developments in pseudophakic dysphotopsia", CURRENT OPINION IN OPHTHALMOLOGY, PHILADELPHIA, PA, US, vol. 17, no. 1, 1 February 2006 (2006-02-01), pages 27-30, XP009189344, ISSN: 1040-8738
- KINNEAR ET AL: "Proposals for scoring and assessing the 100-HUE test", VISION RESEARCH, ELSEVIER, AMSTERDAM, NL, vol. 10, no. 5, 1 May 1970 (1970-05-01), pages 423-433, XP024310971, ISSN: 0042-6989, DOI: 10.1016/0042-6989(70)90123-9 [retrieved on 1970-05-01]

## Description

### TECHNICAL FIELD

This invention relates to a lens assessment device for quantitatively assessing a lens of a patient (examinee) who received a treatment for enlarging a clear vision region, a control program and a control method of the lens assessment device.

### BACKGROUND ART

In the past, a treatment for enlarging a clear vision region has been done on a patient of the cataract, a patient with presbyopia and a patient of the ametropia. For instance, there is a method of such an operation that the lens of the patient is extracted and a multi-focal intraocular lens is inserted in the eye of the patient (see patent related document 1).

### [PRIOR ART]

### [PATENT RELATED DOCUMENT]

[Patent related document 1]: Japanese patent publication No. JP 2006-519031 A relates to a multifocal ophthalmic lens.
[Patent related document 2] : M.-Ch. Lalanne et al., "Pseudophakia and flying personnel aptitude," J. Fr. Ophtaimol. 22(3), pp. 353-358 (1999).
[Patent related document 3]: P. R. Kinnear & A. Sahraie, "New Farnsworth-Munsell 100 hue test norms of normal observers for each year of age 5-22 and for age decades 30-70," Br. J. Ophthalmol. 86, pp. 1408-141 (2002).
[Patent related document 4]: Schwiegerling, "Recent developments in pseudophakic dysphotopsia," Curr. Opin. Ophthalmol. 17, pp. 27-30 (2006).
[Patent related document 5]: P. R. Kinnear, "Proposals for scoring and assessing the 100-HUE test," Vision Res. 10, pp. 423-433 (1970).
[Patent related document 6]: United States patent publication No. US 6, 203, 157 B1 represents the closest prior art and discloses a method for improving accuracy of the visual function testing and reducing human resources in testing the visual function of an examinee.

### [SUMMARY OF INVENTION]

### [PROBLEMS TO BE SOLVED BY INVENTION]

However, a judgment whether a postoperative progress is good exclusively depends on the patient' s judgment, and there is no way of assessing effects of the operation objectively and appropriately. If an intraocular lens is inserted into the eye of the patient through an operation, in some cases the patient feels a sense of incongruity till the patient is used to the intraocular lens. On the other hand, if a diffraction type of the multi-focal intraocular lens is inserted into the eye for the cataract treatment, some patients feel "blur" called Waxy vision, and there is such a problem that it is not easy to judge whether the patient is left unattended since the patient oneself only temporally feels the sense of incongruity or whether it is necessary to exchange the intraocular lens due to Waxy Vision.

An object of the invention is to provide a lens assessment device, a control program and a control method of the lens assessment device for solving the above-mentioned problems. The object is solved by providing a lens assessment device according to claim 1, a control program according to claim 8 and a control method of the lens assessment device according to claim 14. Further embodiments of the invention are defined in the respective dependent claims.

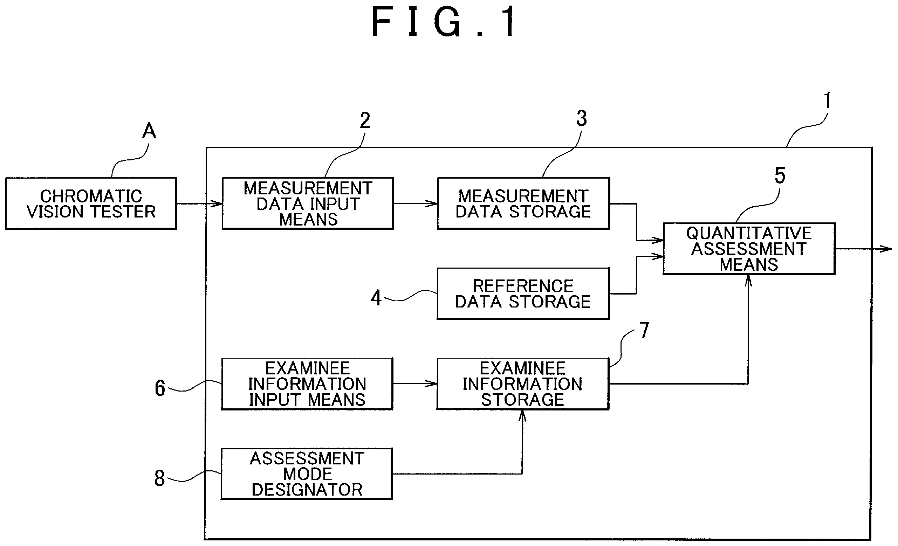

An aspect of the invention, which is exemplarily shown in Fig.1, relates to a lens assessment device (1) for quantitatively assessing a lens of an examinee who received a treatment for enlarging a clear vision region, comprising:
measurement data input means (2) for inputting measurement data on a chromatic vision of the examinee;
a measurement data storage (3) for storing inputted measurement data;
a reference data storage (4) for storing reference data on the chromatic vision; and
quantitative assessment means (5) for quantitatively assessing the lens of the examinee by comparing the measurement data and the reference data.

In an embodiment of the invention, the lens is an artificial lens inserted into an eye of the examinee through an operation or an original lens that is not the artificial lens.

In an embodiment of the invention, the reference data storage (4) stores at least any of the measurement data of chromatic vision of a large group of people who do not install the artificial lens, and the measurement data of chromatic vision of a large group of people who install the artificial lens and classified by kinds of the artificial lenses as the reference data.

In an embodiment of the invention, the reference data storage (4) stores the measurement data of chromatic vision, classified by ages.

In an embodiment of the invention, the reference data storage (4) stores the data classified by kinds of the artificial lenses, further classified by postoperative elapsed time.

In an embodiment of the invention, the lens assessment device further comprises:
examinee information input means (6) for inputting at least one data of kinds of the lenses of the examinees, ages of examinees and postoperative elapsed time as examinee information;
examinee information storage (7) for storing the inputted examinee information;
assessment mode designator (8) for designating with which examinee information of the examinee information stored in the examinee information storage (7) and with which reference data of the reference data stored in the reference data storage (4) quantitative assessment should be done.

In an embodiment of the invention, the quantitative assessment means (5) compares the measurement data on specific colors that are easy to receive an influence according to the kind of the lens, the age of the examinee or said postoperative elapsed time designated through the assessment mode designator (8).

Another aspect of the invention relates to a control program for controlling a computer, the control program providing a computer to function as follows:
measurement data input means (2) for inputting measurement data of a chromatic vision of an examinee;
a measurement data storage (3) for storing the inputted measurement data;
a reference data storage (4) for storing reference data on the chromatic vision; and
quantitative assessment means (5) for quantitatively assessing a lens of the examinee by comparing the measurement data and the reference data.

In an embodiment of the invention, the reference data storage (4) stores at least any of measurement data of the chromatic vision of a large group of people who do not install an artificial lens, measurement data of the chromatic vision of a large group of people who install the artificial lens and classified by kinds of the artificial lenses as the reference data.

In an embodiment of the invention, the reference data storage (4) stores the measurement data of the chromatic vision, classified by ages.

In an embodiment of the invention, the reference data storage (4) stores the data classified by kinds of the artificial lenses, further classified by postoperative elapsed time.

In an embodiment of the invention, the control program further providing the computer to function as follows:
an examinee information input means (6) for inputting at least one data of kinds of the lenses of the examinees, ages of examinees and postoperative elapsed time as examinee information;
an examinee information storage (7) for storing the inputted examinee information;
an assessment mode designator (8) for designating with which examinee information of the examinee information stored in the examinee information storage (7) and with which reference data of the reference data stored in the reference data storage (4) quantitative assessment should be done.

In an embodiment of the invention, the quantitative assessment means (5) compares measurement data on specific colors that are easy to receive an influence according to the kind of the lens, the age of the examinee or the postoperative elapsed time designated through the assessment mode designator (8).

Another aspect of the invention relates to a control method of a lens assessment device (1) for quantitatively assessing a lens of an examinee who received a treatment for enlarging a clear vision region, comprising:
a step of inputting measurement data on a chromatic vision of an examinee through measurement data input means (2);
a step of storing the inputted measurement data through a measurement data storage (3);
a step of storing reference data on chromatic vision through a reference data storage (4); and
a step of quantitatively assessing the lens of the examinee through quantitative assessment means (5) by comparing the measurement data and the reference data.

In an embodiment of the invention, the reference data storage (4) stores at least any of measurement data of the chromatic vision of a large group of people who do not install an artificial lens, and measurement data of the chromatic vision of a large group of people who install the artificial lens and classified by kinds of the artificial lenses as the reference data.

In an embodiment of the invention, said reference data storage (4) stores the measurement data of the chromatic vision, classified by ages.

In an embodiment of the invention, the reference data storage (4) stores the data classified by kinds of the artificial lenses, further classified by postoperative elapsed time.

In an embodiment of the invention, the control method of the lens assessment device further comprises:
a step of inputting at least one data of kinds of the lenses of the examinees, ages of the examinees and postoperative elapsed time as examinee information through examinee information input means (6);
a step of storing the inputted examinee information through examinee information storage (7); and
a step of designating with which examinee information of the examinee information stored in the examinee information storage (7) and with which reference data of the reference data stored in the reference data storage (4) quantitative assessment should be done through assessment mode designator (8).

In an embodiment of the invention, said quantitative assessment means (5) compares the measurement data on specific colors that are easy to receive an influence according to the kinds of the lenses, the ages of the examinees or the postoperative elapsed time designated through the assessment mode designator (8).

The number in parentheses shows the corresponding element in the drawings for the sake of convenience, and accordingly, the descriptions are not restricted and bound by the descriptions on the drawings.

### EFFECTS OF INVENTION

According to the inventions of claims 1, 2, 3, 8, 9, 14 and 15, the chromatic vision of the examinee who received a treatment for widening the clear vision area is measured, and the measurement data is quantitatively compared with predetermined reference data (the reference data of the chromatic vision), so that it is possible to appropriately judge whether or not postoperative progress is good. If the artificial lens was inserted in the eye of the examinee through an operation, it is possible to properly objectively judge whether or not the artificial lens is proper. Therefore, if the diffraction type of multifocal intraocular lens was inserted in an eye of the examinee for cataract treatment and the examinee feels a sense of incongruity, it is possible to objectively judge whether the examinee only feels temporary sense of incongruity and such a situation should be left as it is, or whether it is necessary to exchange the intraocular lens due to occurrence of Waxy Vision. Furthermore, it is possible to give objective and proper information to the person who wishes to receive an operation for widening the clear vision region by accumulating such objective assessment as data.

According to the inventions of claims 4, 5, 10, 11, 16 and 17, it is possible to properly judge as to whether the postoperative progress is good by taking the age of the examinee or the postoperative elapsed time into consideration.

According to the inventions of claims 6, 12 and 18, if the examiner only designates the assessment mode through the assessment mode designator, the quantitative assessment means appropriately reads necessary information or data out of the measurement data storage, the reference data storage and the examinee information storage so as to do quantitative assessment, and such effects that the operation is made simple are exercised.

According to the inventions of claims 7, 13 and 19, a simple assessment in a short time is possible in comparison with a case of assessment on all colors .

### BRIEF DESCRIPTION OF DRAWINGS

[Fig.1] Fig.1 is a block diagram showing an instance of a structure of a lens assessment device according to the invention.
[Fig.2] Fig.2 is a view showing an instance of measurement data of color vision.
[Fig.3] Fig.3(a) is a view showing a general tendency of the color vision of persons in their 30s, Fig.3(b) is a view showing a general tendency of the color vision of persons in their 40s, Fig.3(c) is a view showing a general tendency of the color vision of persons in their 50s.
[Fig.4] Fig.4 is a view showing a general tendency of the color vision of persons in their 50s and 60s who installed a multifocal intraocular lens.
[Fig.5] Fig.5 is a block diagram showing an instance of a structure of a chromatic vision tester.

### EMBODIMENTS FOR CARRYING OUT THE INVENTION

An embodiment of the invention is now explained, referring to Figs.1 to 5.

A lens assessment device according to the invention quantitatively assesses a lens of a patient (examinee) who received a treatment for enlarging a clear vision region. In the specification, the lenses are artificial lenses inserted to be the examinees through the operations for enlarging the clear vision region, concretely well-known intraocular lenses, such as an intraocular lens to be inserted for the treatment of cataract (multifocal intraocular lens), an intraocular lens for correcting astigmatism and an intraocular lens for correcting presbyopia, but original lenses (lens that received a treatment for enlarging the clear vision region without inserting the intraocular lens) that are not artificial lenses are not always excluded.

A lens assessment device according to the invention shown with a reference number 1 in Fig.1 has a measurement data input means 2 through which measurement data of an examinee on color vision is inputted, a measurement data storage 3 for storing the inputted measurement data, a reference data storage 4 that stores reference data on the color vision and a quantitative assessment means 5 that quantitatively assesses the lens of the examinee by comparing the measurement data and the reference data with each other.

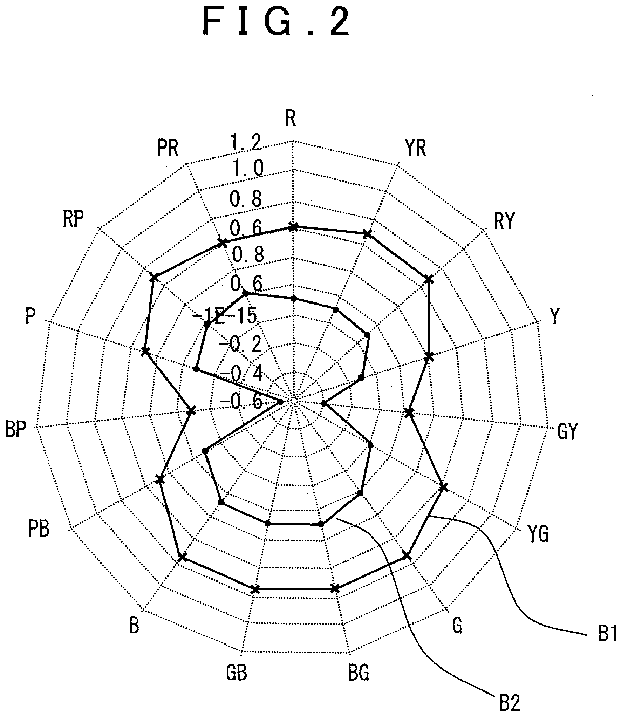

The above-mentioned measurement data and the reference data both relate to measurements of chromatic vision, and to measurements of the visual acuity of respective colors R, YR, RY, Y, GY, ... as shown in Fig.2. A reference number B1 denotes measurement data of a patient in which a multifocal intraocular lens was inserted, and a reference number B2 denotes measurement data of a patient in which a single-focus intraocular lens was inserted. Preferably, such measurement of chromatic vision is conducted through a chromatic vision tester shown with a reference number A in Fig.1. A structure of such a tester is mentioned hereinafter.

The above-mentioned measurement data input means 2 are a keyboard or a touch panel (through which measurement data is inputted by an examiner) , a mouse (through which measurement data is inputted while the examiner is watching an user interface on a screen) and an I/O interface for connecting a memory medium, such as a USB memory and a SD card (the measurement data stored in the memory medium through the above-mentioned chromatic vision tester is automatically inputted in the above-mentioned measurement data storage 3 through connection of the memory medium).

Furthermore, a method of quantitatively assessing the lens of the examinee through the above-mentioned quantitative assessment means 5 is a method of assessing for respective colors (R, YR, RY, Y, GY, ...) by digitizing a relation between the measurement data and the reference data, such as a fraction, a decimal and percentage. Concretely, the methods are the method of making a ratio between the measurement data and the reference data a fraction or a decimal so as to assess (for instance, the method of assessing with 0.4/0.6 if the reference data of some color is 0.6 and the measurement data is 0.4) and the method of assessing a difference between the reference data and the measurement data as the ratio with respect to the reference data , such as a fraction, a decimal and a rate. Such numerals may be shown on a display or on a paper, or a ranking (characters, such as a rank, b rank and c rank) based upon the numerals may be shown on the display or the paper.

According to the invention, the chromatic vision of the examinee who received a treatment for widening the clear vision region is measured, and the measurement data is quantitatively compared with predetermined reference data (the reference data of the chromatic vision), so that it is possible to appropriately judge whether or not postoperative progress is good. If the artificial lens was inserted in the eye of the examinee through an operation, it is possible to properly objectively judge whether or not the artificial lens is proper. Therefore, if the diffraction type of multifocal intraocular lens is inserted in an eye of the examinee for cataract treatment and the examinee feels a sense of incongruity, it is possible to objectively judge whether the examinee only feels temporary sense of incongruity and such a situation should be left as it is, or whether it is necessary to exchange the intraocular lens due to occurrence of Waxy Vision. Furthermore, it is possible to give objective and proper information to the person who wishes to receive an operation for widening a clear vision region by accumulating such objective assessment as data.

Preferably, the reference data storage 4 stores at least one of both data, measurement data of the chromatic vision of many persons who do not install the artificial lens (that is, the measurement data of many normal persons in connection with the chromatic vision, and the measurement data of many persons who installs the artificial leans, that are classified for respective kinds of the artificial lenses. Preferably, the measurement data on the chromatic vision is stored, classified by ages. Furthermore, the data classified by the respective kinds of the artificial lenses may be stored, being classified every postoperative elapsed time. For instance, when considering three kinds of artificial lenses ("A intraocular lens", "B intraocular lens" and "C intraocular lens") and the lenses (original lens that is not the artificial lens), preferably the reference data storage 4 stores reference data, that is, the reference data in connection with the lens (classified by ages), the reference data in connection with the lens (not classified by ages), the reference data of A intraocular lens (classified by ages and by postoperative elapsed time), the B intraocular lens (classified by ages and by postoperative elapsed time), the reference data of C intraocular lens (classified by ages and by postoperative elapsed time), the reference data of A intraocular lens (classified by ages and not classified by postoperative elapsed time), the reference data of B intraocular lens (classified by ages and not classified by postoperative elapsed time), the reference data of C intraocular lens (classified by ages and not classified by postoperative elapsed time), the reference data of A intraocular lens (not classified by ages and not classified by postoperative elapsed time), the reference data of B intraocular lens (not classified by ages and not classified by postoperative elapsed time), and the reference data of C intraocular lens (not classified by ages and not classified by postoperative elapsed time) (all of the reference data or a part thereof).

It is possible to properly judge whether the postoperative progress is good when assessing with the reference data classified by ages and the reference data classified by the postoperative elapsed time. For instance, it is not possible to deny a fear to accompany complications such as edema if the intraocular lens was inserted, but it is possible to early find concurrence of such complications through quantitative assessment in consideration of the above-mentioned postoperative elapsed time.

On the other hand, preferably the lens assessment device 1 according to the invention has an examinee information input means 6 through which at least one of data, such as kinds of the lenses of the examinees (that is, information whether it is the artificial lens that inserted into the eye of the patient through the operation or an original lens that is not the artificial lens, or type number or article name if it is the artificial lens), ages of the examinees, the postoperative elapsed time if the artificial lens was inserted through the operation (number of elapsed days and number of elapsed months), is inputted as examinee information, an examinee information storage 7 for storing inputted examinee information, and an assessment mode designator 8 for designating with which examinee information of the examinee information stored in the examinee information storage and with which reference data stored in the reference data storage the quantitative assessment should be done. In such a case, if the examiner designates the assessment mode (that is, with which examinee information and with which reference data the quantitative assessment should be done) through the assessment mode designator 8, the quantitative assessment means 5 reads necessary examinee information out of the examinee information storage 7, and reads the reference data corresponding thereto out of the reference data storage and compares the read data with the measurement data. In such a case, if the examiner only designates the assessment mode through the assessment mode designator 8, the quantitative assessment means 5 appropriately reads necessary information or data out of the measurement data storage 3, the reference data storage 4 and the examinee information storage 7 so as to do quantitative assessment, and such effects that the operation is made simple are exercised.

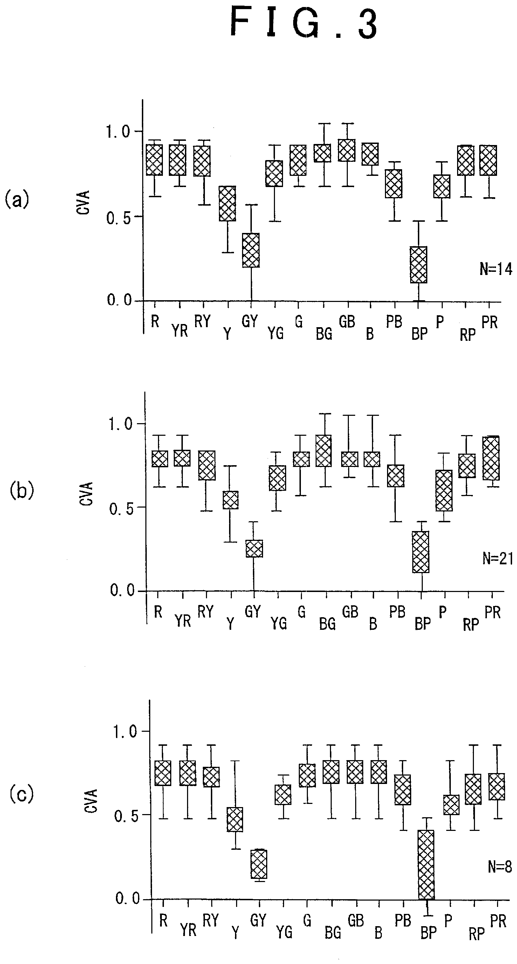

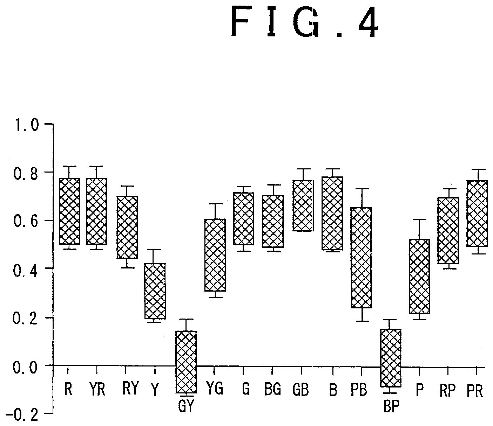

Preferably, the chromatic vision of only specific color is measured and is quantitatively assessed without measuring the chromatic vision of all color regions although the chromatic vision for all color regions (R, YR, RY, Y, GY, YG, G, BG, GB, B, PB, BP, P, RP, PR) was measured in an instance of Fig.2). For instance, the chromatic vision of persons in their 30s has an inclination as shown in Fig.3(a), the chromatic vision of persons in their 40s has an inclination as shown in Fig.3(b), the chromatic vision of persons in their 50s has an inclination as shown in Fig.3(c), and the chromatic vision of persons who install the multifocal intraocular lens (persons in their 50s and 60s) has an inclination as shown in Fig.4. Then, it is possible to assess the examinee (the lens) only by measuring the chromatic vision of a specific color (the color that is easy to receive an influence by an operation) without measuring the chromatic vision of all colors. Preferably, the quantitative assessment means 5 compares the measurement data of the specific colors that are easy to receive an influence according to the kind of the lens, the age of the examinee or the postoperative elapsed time designated by the assessment mode designator 8. In such a case, simple assessment in a short time is possible in comparison with a case of the assessment on all colors.

On the other hand, the examinee information input means 6 are a keyboard and a touch panel (through which data, such as ages of the examinees, are inputted by operating it by the examiner), a mouse (through which data are inputted by the examiner while the examiner is watching a user interface on a screen), and an I/O interface for connecting a storage medium, such as an USB memory and a SD card, (through which data are automatically inputted by connection of the storage medium). And, the above-mentioned assessment mode designator 8 are a keyboard and a touch panel (through which data, such as ages of the examinees, are inputted by operating it by the examiner), a mouse (through which data are inputted by the examiner while the examiner is watching a user interface on a screen) and well-known various switches. Preferably, the keyboard and the like are used also as the above-mentioned measurement data input means.

One of the above-mentioned chromatic vision tester A is a device for displaying two or more test images on a color monitor, switching in order. One of such test images is the image showing some stimulus, such as a character, a numeral, a Landolt ring and the other graphics therein. The color monitors are liquid crystal display and the other type of monitors, such as a CRT and a plasma display).

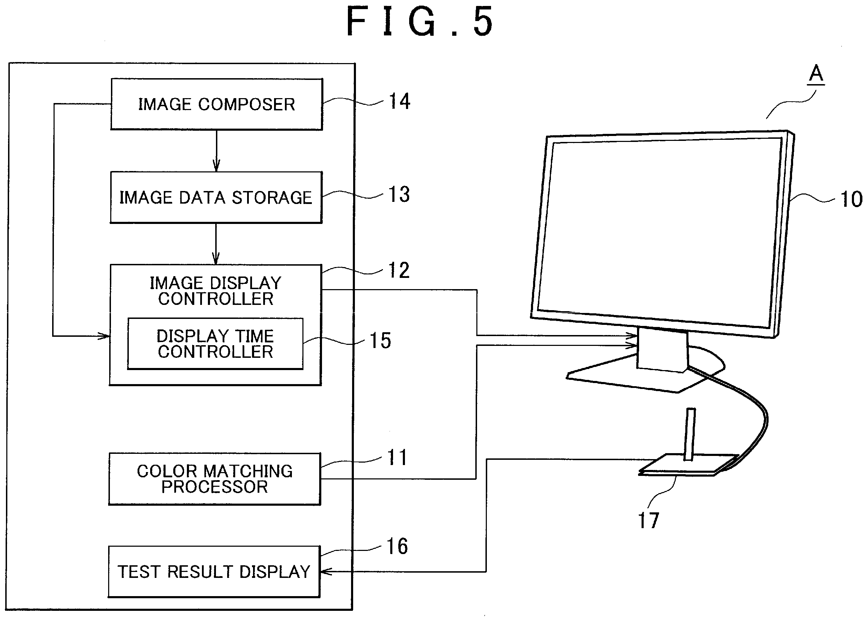

As shown in detail in Fig.5, the chromatic vision tester A has a color monitor 10 for presenting the test images, a color matching processor 11 for doing a color matching processing on the color monitor 10, an image display controller 12 for executing at least one display step wherein two or more test images are displayed on the color monitor on which the color matching processing was done. And, the image display controller 12 changes any one ("the first element" hereinafter) of six elements, a hue of each test image, a brightness of each test image, a saturation of each test image and a display time of each test image and a brightness of a background or a stimulus if the test image is comprised of a background image (the background image in an optional color) and the stimulus shown in the background image, and fixes five elements excluding the first element when displaying two or more test images in the one display step. In such a case, the color matching processing is a processing for maintaining color identity so as not to occur a change with passage of time on the color displayed through the color monitor. Preferably, the above-mentioned color matching processor 11 is comprised of a sensor portion (not shown) for detecting the color displayed through the color monitor 10 and digitizing it and a color corrector (not shown) for correcting the color displayed through the color monitor 10 by feedback control with numerals from the sensor portion.

An image data storage 13 in Fig.5 is means for storing the test images to be displayed on the color monitor 10, an image composer 14 is means for composing the test image to be displayed by the examiner, a display time controller 15 is means for controlling the display time of each test image, and a test result display 16 is means for displaying a result as to whether the examinee can perceive the test image, a kind of the displayed test image, information on combination of colors used in the displayed test image and the display time of the test image. And, a reference 17 in Fig.5 denotes a joystick for inputting a result as to whether the examinee perceived the test image by the examinee himself.

On the other hand, a control program according to the invention is for controlling a computer, and for getting the computer to function as the measurement data input means 2 for inputting the measurement data on the chromatic vision of the examinees, the measurement data storage 3 for storing the inputted measurement data, the reference data storage 4 for storing reference data in connection with the chromatic vision, and the quantitative assessment means 5 for quantitatively assessing the lens of the examinee by comparing the measurement data and the reference data.

Preferably, in such a case, the reference data storage 4 stores any one of the measurement data of the chromatic vision on a large group of people who do not install the artificial lens and the measurement data of the chromatic vision on a large group of people who install the artificial lens that are classified by the kinds of the artificial lenses, as the reference data. Preferably, the reference data storage 4 stores the measurement data of the chromatic vision, classified by ages. Furthermore, preferably the reference data storage 4 stores the data classified by the kinds of the artificial lenses, further classified by the postoperative elapsed time.

Preferably, the control program gets the computer to function as the examinee information input means 6 for inputting at least one data of the kind of the lens of the examinee, the age of the examinee and the postoperative elapsed time as the examinee information, the examinee information storage 7 for storing the inputted examinee information, and the assessment mode designator 8 for designating with which examinee information of the examinee information stored in the examinee information storage 7 and with which reference data of the reference data stored in the reference data storage 3 the quantitative assessment should be done.

Preferably the control program gets the quantitative assessment means 5 to compare the measurement data in connection with the specific colors that are easy to receive an influence according to the kind of the lens, the age of the examinee or the postoperative elapsed time designated by the assessment mode designator 8.

On the other hand, a control method of the lens assessment device according to the invention is for quantitatively assessing the lens of the examinee who received the treatment for enlarging the clear vision region, and has a step of inputting the measurement data of the chromatic vision of the examinee through the measurement data input means 2, a step of storing the inputted measurement data through the measurement data storage 3, a step of storing the reference data of the chromatic vision through the reference data storage 4, and a step of quantitatively assessing the lens of the examinee by comparing the measurement data and the reference data through the quantitative assessment means 5.

Preferably, in such a case, the reference data storage 4 stores at least any of the measurement data of the chromatic vision of a large group of people who do not install the artificial lens and the measurement data of the chromatic vision of a large group of people who install the artificial lens that is classified by the kinds of the artificial lenses. Preferably, the reference data storage 4 stores the measurement data of chromatic vision, classified by ages. Besides, the reference data storage 4 stores the data classified by kinds of the artificial lenses, further classified by postoperative elapsed time.

Preferably, the control method has a step of inputting at least one data of kinds of the lenses of the examinees, ages of examinees and the postoperative elapsed time as examinee information through examinee information input means 6, a step of storing the inputted examinee information through the examinee information storage 7, and a step of designating with which examinee information of the examinee information stored in the examinee information storage 7 and with which reference data of the reference data stored in the reference data storage 4 quantitative assessment should be done through assessment mode designator 8.

Preferably, the quantitative assessment means 5 compares the measurement data on the specific colors that are easy to receive an influence according to the kind of the lens, the age of the examinee or the postoperative elapsed time designated by the assessment mode designator 8.

### EXPLANATION OF REFERENCE NUMBERS

- 1: lens assessment device
- 2: measurement data input means
- 3: measurement data storage
- 4: reference data storage
- 5: quantitative assessment means
- 6: examinee information input means
- 7: examinee information storage
- 8: assessment mode designator
- A: chromatic vision tester

## Claims

1. A lens assessment device (1) for quantitatively assessing a lens of an examinee, comprising:
measurement data input means (2) for inputting measurement data on a chromatic vision of the examinee;
a measurement data storage (3) for storing the inputted measurement data;
a reference data storage (4) for storing reference data on the chromatic vision; and
quantitative assessment means (5) for quantitatively assessing the lens of the examinee by comparing the measurement data and the reference data,
wherein the measurement data and the reference data both relate to measurement data of the chromatic vision for one or more specific colors being measured based on two or more test images displayed on a color monitor, one of which is an image showing a stimulus, and
wherein the reference data storage (4) stores data as the reference data, the data being measurement data of chromatic vision of a large group of people who have an artificial lens inserted into the eye and the data being classified by kinds of the artificial lenses.

2. The lens assessment device (1) according to claim 1, wherein the lens is an artificial lens inserted into an eye of the examinee through an operation or an original lens that is not the artificial lens.

3. The lens assessment device (1) according to claim 1 or 2, wherein the reference data storage (4) further stores measurement data of chromatic vision of a large group of people who do not install an artificial lens as the reference data.

4. The lens assessment device (1) according to claim 1, wherein the reference data storage (4) stores the measurement data of chromatic vision, classified by ages.

5. The lens assessment device (1) according to claim 1 or 4, wherein the reference data storage (4) stores the data classified by kinds of the artificial lenses, further classified by postoperative elapsed time.

6. The lens assessment device (1) according to any of claims 1 to 5, further comprising:
examinee information input means (6) for inputting at least one data of kinds of the lenses of the examinees, ages of examinees and postoperative elapsed time as examinee information;
examinee information storage (7) for storing the inputted examinee information;
assessment mode designator (8) for designating with which examinee information of the examinee information stored in the examinee information storage (7) and with which reference data of the reference data stored in the reference data storage (4) quantitative assessment should be done.

7. The lens assessment device (1) according to claim 6, wherein the quantitative assessment means (5) compares the measurement data on two different colors according to the kind of the lens, the age of the examinee or the postoperative elapsed time designated through the assessment mode designator (8).

8. A control program for controlling a computer to function as follows:
measurement data input means (2) for inputting measurement data of a chromatic vision of an examinee;
measurement data storage (3) for storing the inputted measurement data;
reference data storage (4) for storing reference data on the chromatic vision; and
quantitative assessment means (5) for quantitatively assessing a lens of the examinee by comparing the measurement data and the reference data,
wherein the measurement data and the reference data both relate to measurement data of the chromatic vision for one or more specific colors being measured based on two or more test images displayed on a color monitor, one of which is an image showing a stimulus, and
wherein the reference data storage (4) stores data as the reference data, the data being measurement data of chromatic vision of a large group of people who have an artificial lens inserted into the eye and the data being classified by kinds of the artificial lenses.

9. The control program according to claim 8, wherein the reference data storage (4) further stores measurement data of the chromatic vision of a large group of people who do not install an artificial lens as the reference data.

10. The control program according to claim 8, wherein the reference data storage (4) stores the measurement data of the chromatic vision, classified by ages.

11. The control program according to claim 8 or 10, wherein the reference data storage (4) stores the data classified by kinds of the artificial lenses, further classified by postoperative elapsed time.

12. The control program according to any of claims 8 to 11, for providing the computer to further function as follows:
examinee information input means (6) for inputting at least one data of kinds of the lenses of the examinees, ages of examinees and postoperative elapsed time as examinee information;
an examinee information storage (7) for storing the inputted examinee information;
an assessment mode designator (8) for designating with which examinee information of the examinee information stored in the examinee information storage (7) and with which reference data of the reference data stored in the reference data storage (4) quantitative assessment should be done.

13. The control program according to claim 12, wherein the quantitative assessment means (5) compares measurement data on two different colors according to the kind of the lens, the age of the examinee or the postoperative elapsed time designated through the assessment mode designator (8).

14. A control method of a lens assessment device (1) for quantitatively assessing a lens of an examinee, comprising:
a step of inputting measurement data on a chromatic vision of the examinee through measurement data input means (2);
a step of storing the inputted measurement data through a measurement data storage (3);
a step of storing reference data on the chromatic vision through a reference data storage (4); and
a step of quantitatively assessing the lens of the examinee through quantitative assessment means (5) by comparing the measurement data and the reference data,
wherein the measurement data and the reference data both relate to measurement data of the chromatic vision for one or more specific colors being measured based on two or more test images displayed on a color monitor, one of which is an image showing a stimulus, and
wherein the reference data storage (4) stores data as the reference data, the data being measurement data of chromatic vision of a large group of people who have an artificial lens inserted into the eye and the data being classified by kinds of the artificial lenses.

15. The control method of the lens assessment device (1) according to claim 14, wherein the reference data storage (4) further stores measurement data of the chromatic vision of a large group of people who do not install an artificial lens as the reference data.

16. The control method of the lens assessment device (1) according to claim 14, wherein the reference data storage (4) stores the measurement data of the chromatic vision, classified by ages.

17. The control method of the lens assessment device (1) according to claim 14 or 16, wherein the reference data storage (4) stores the data classified by kinds of the artificial lenses, further classified by postoperative elapsed time.

18. The control method of the lens assessment device (1) according to any of claims 14 to 17, further comprising:
a step of inputting at least one data of kinds of the lenses of the examinees, ages of the examinees and postoperative elapsed time as examinee information through examinee information input means (6);
a step of storing the inputted examinee information through examinee information storage (7); and
a step of designating with which examinee information of the examinee information stored in the examinee information storage (7) and with which reference data of the reference data stored in the reference data storage (4) quantitative assessment should be done through assessment mode designator (8).

19. The control method of the lens assessment device (1) according to claim 18, wherein the quantitative assessment means (5) compares the measurement data on two different colors according to the kinds of the lenses, the ages of the examinees or the postoperative elapsed time designated through the assessment mode designator (8).

## Patentansprüche

1. Linsenbeurteilungsvorrichtung (1) zur quantitativen Beurteilung einer Linse einer untersuchten Person, umfassend:
ein Messdateneingabemittel (2) zur Eingabe von Messdaten über das Farbsehen der untersuchten Person;
einen Messdatenspeicher (3) zum Speichern der eingegebenen Messdaten;
einen Referenzdatenspeicher (4) zum Speichern von Referenzdaten über das Farbsehen; und
ein Mittel (5) zur quantitativen Beurteilung zum quantitativen Beurteilen der Linse der untersuchten Person durch Vergleichen der Messdaten mit den Referenzdaten,
wobei sich die Messdaten und die Referenzdaten beide auf Messdaten des Farbsehens für eine oder mehrere spezifische Farben beziehen, die auf der Grundlage von zwei oder mehreren Testbildern gemessen werden, die auf einem Farbmonitor angezeigt werden, von denen eines ein Bild ist, das einen Stimulus zeigt, und
wobei der Referenzdatenspeicher (4) Daten als die Referenzdaten speichert, wobei die Daten Messdaten des Farbsehens einer großen Gruppe von Personen sind, die eine künstliche Linse in das Auge eingesetzt haben, und die Daten nach Arten der künstlichen Linsen klassifiziert sind.

2. Linsenbeurteilungsvorrichtung (1) nach Anspruch 1, wobei die Linse eine künstliche Linse ist, die durch eine Operation in ein Auge der untersuchten Person eingesetzt wird, oder eine ursprüngliche Linse, die nicht die künstliche Linse ist.

3. Linsenbeurteilungsvorrichtung (1) nach Anspruch 1 oder 2, wobei der Referenzdatenspeicher (4) ferner Messdaten des Farbsehens einer großen Gruppe von Personen, die keine künstliche Linse einsetzen, als Referenzdaten speichert.

4. Linsenbeurteilungsvorrichtung (1) nach Anspruch 1, wobei der Referenzdatenspeicher (4) die Messdaten des Farbsehens, klassifiziert nach dem Alter, speichert.

5. Linsenbeurteilungsvorrichtung (1) nach Anspruch 1 oder 4, wobei der Referenzdatenspeicher (4) die Daten klassifiziert nach Art der künstlichen Linsen, ferner klassifiziert nach postoperativ verstrichener Zeit, speichert.

6. Linsenbeurteilungsvorrichtung (1) nach einem der Ansprüche 1 bis 5, ferner umfassend:
Mittel zur Eingabe von Informationen (6) über die untersuchte Person zur Eingabe von mindestens einer der folgenden Daten: Art der Linsen der untersuchten Personen, Alter der untersuchten Personen und postoperativ verstrichene Zeit als Informationen über die untersuchte Person;
Speicher (7) für Informationen über die untersuchte Person zur Speicherung der eingegebenen Informationen über die untersuchte Person;
einen Beurteilungsmodusbezeichner (8) zur Bezeichnung, mit welchen Informationen über die untersuchte Person der im Speicher (7) für Informationen über die untersuche Person gespeicherten Informationen über die untersuchte Person und mit welchen Referenzdaten der im Referenzdatenspeicher (4) gespeicherten Referenzdaten eine quantitative Beurteilung durchgeführt werden soll.

7. Linsenbeurteilungsvorrichtung (1) nach Anspruch 6, wobei das Mittel (5) zur quantitativen Beurteilung die Messdaten in zwei verschiedenen Farben vergleicht, je nach Art der Linse, dem Alter der untersuchten Person oder der postoperativ verstrichenen Zeit, die durch den Beurteilungsmodusbezeichner (8) bestimmt werden.

8. Steuerprogramm zum Steuern eines Computers, um wie folgt zu funktionieren:
ein Messdateneingabemittel (2) zum Eingeben von Messdaten des Farbsehens einer untersuchten Person;
Messdatenspeicher (3) zum Speichern der eingegebenen Messdaten;
Referenzdatenspeicher (4) zum Speichern von Referenzdaten über das Farbsehen; und
ein Mittel (5) zur quantitativen Beurteilung einer Linse der untersuchten Person durch Vergleichen der Messdaten und der Referenzdaten,
wobei sich die Messdaten und die Referenzdaten beide auf Messdaten des Farbsehens für eine oder mehrere spezifische Farben beziehen, die auf der Grundlage von zwei oder mehreren Testbildern gemessen werden, die auf einem Farbmonitor angezeigt werden, von denen eines ein Bild ist, das einen Stimulus zeigt, und
wobei der Referenzdatenspeicher (4) Daten als die Referenzdaten speichert, wobei die Daten Messdaten des Farbsehens einer großen Gruppe von Personen sind, die eine künstliche Linse in das Auge eingesetzt haben, und die Daten nach Arten der künstlichen Linsen klassifiziert sind.

9. Steuerprogramm nach Anspruch 8, wobei der Referenzdatenspeicher (4) ferner Messdaten des Farbsehens einer großen Gruppe von Personen, die keine künstliche Linse einsetzen, als Referenzdaten speichert.

10. Steuerprogramm nach Anspruch 8, wobei der Referenzdatenspeicher (4) die Messdaten des Farbsehens, klassifiziert nach dem Alter, speichert.

11. Steuerprogramm nach Anspruch 8 oder 10, wobei der Referenzdatenspeicher (4) die Daten klassifiziert nach Art der künstlichen Linsen , weiter klassifiziert nach postoperativ verstrichener Zeit, speichert.

12. Steuerprogramm nach einem der Ansprüche 8 bis 11, das dem Computer die folgenden weiteren Funktionen ermöglicht:
ein Mittel (6) zur Eingabe von Informationen über die untersuchte Person zur Eingabe von mindestens einer der folgenden Daten: Art der Linsen der untersuchten Personen, Alter der untersuchten Personen und postoperativ verstrichene Zeit als Informationen über die untersuchte Person;
einen Speicher (7) für Informationen über die untersuchte Person zum Speichern der eingegebenen Informationen über die untersuchte Person;
einen Beurteilungsmodusbezeichner (8) zur Bezeichnung, mit welchen Informationen über die untersuchte Person der im Speicher (7) für Informationen über die untersuchte Person gespeicherten Informationen über die untersuchte Person und mit welchen Referenzdaten der im Referenzdatenspeicher (4) gespeicherten Referenzdaten eine quantitative Beurteilung durchgeführt werden soll.

13. Steuerprogramm nach Anspruch 12, wobei das Mittel (5) zur quantitativen Beurteilung (5) die Messdaten in zwei verschiedenen Farben vergleicht, je nach Art der Linse, dem Alter der untersuchten Person oder der postoperativ verstrichenen Zeit , die durch den Beurteilungsmodusbezeichner (8) bestimmt werden.

14. Verfahren zur Steuerung einer Linsenbeurteilungsvorrichtung (1) zur quantitativen Beurteilung einer Linse einer untersuchten Person, umfassend:
einen Schritt der Eingabe von Messdaten über das Farbsehen der untersuchten Person durch ein Messdateneingabemittel (2);
einen Schritt zum Speichern der eingegebenen Messdaten in einem Messdatenspeicher (3);
einen Schritt der Speicherung von Referenzdaten über das Farbsehen durch einen Referenzdatenspeicher (4); und
einen Schritt der quantitativen Beurteilung der Linse der untersuchten Person durch ein Mittel (5) zur quantitativen Beurteilung durch Vergleichen der Messdaten und der Referenzdaten,
wobei sich die Messdaten und die Referenzdaten beide auf Messdaten des Farbsehens für eine oder mehrere spezifische Farben beziehen, die auf der Grundlage von zwei oder mehreren Testbildern gemessen werden, die auf einem Farbmonitor angezeigt werden, von denen eines ein Bild ist, das einen Stimulus zeigt, und
wobei der Referenzdatenspeicher (4) Daten als die Referenzdaten speichert, wobei die Daten Messdaten des Farbsehens einer großen Gruppe von Personen sind, die eine künstliche Linse in das Auge eingesetzt haben, und die Daten nach Arten der künstlichen Linsen klassifiziert sind.

15. Verfahren zur Steuerung der Linsenbeurteilungsvorrichtung (1) nach Anspruch 14, wobei der Referenzdatenspeicher (4) ferner Messdaten des Farbsehens einer großen Gruppe von Personen, die keine künstliche Linse einsetzen, als Referenzdaten speichert.

16. Verfahren zur Steuerung der Linsenbeurteilungsvorrichtung (1) nach Anspruch 14, wobei der Referenzdatenspeicher (4) die Messdaten des Farbsehens, klassifiziert nach Alter, speichert.

17. Verfahren zur Steuerung der Linsenbeurteilungsvorrichtung (1) nach Anspruch 14 oder 16, wobei der Referenzdatenspeicher (4) die Daten klassifiziert nach Art der künstlichen Linsen, ferner klassifiziert nach postoperativ verstrichener Zeit, speichert.

18. Verfahren zur Steuerung der Linsenbeurteilungsvorrichtung (1) nach einem der Ansprüche 14 bis 17, das ferner Folgendes umfasst:
einen Schritt des Eingebens von mindestens einer der folgenden Daten: Art der Linsen der untersuchten Personen, Alter der untersuchten Personen und postoperativ verstrichene Zeit als Informationen über die untersuchte Person durch das Mittel (6) zur Eingabe von Informationen über die untersuchte Person ;
einen Schritt des Speicherns der eingegebenen Informationen über die untersuchte Person durch einen Speicher (7) für Informationen über die untersuchte Person; und
einen Schritt des Bezeichnens, mit welchen Informationen über die untersuchte Person der im Speicher (7) für Informationen über die untersuchte Person gespeicherten Informationen über die untersuchte Person und mit welchen Referenzdaten der im Referenzdatenspeicher (4) gespeicherten Referenzdaten eine quantitative Beurteilung durch einen Beurteilungsmodusbezeichner (8) durchgeführt werden soll.

19. Verfahren zur Steuerung der Linsenbeurteilungsvorrichtung (1) nach Anspruch 18, wobei das Mittel zur quantitativen Beurteilung (5) die Messdaten in zwei verschiedenen Farben vergleicht, je nach Art der Linsen, dem Alter der untersuchten Personen oder der postoperativ verstrichenen Zeit, die durch den Beurteilungsmodusbezeichner (8) bestimmt werden.

## Revendications

1. Dispositif d'évaluation de lentille (1) permettant d'évaluer quantitativement la lentille d'une personne examinée, comprenant :
un moyen d'entrée de données de mesure (2) pour entrer des données de mesure sur la vision chromatique de la personne examinée ;
une mémoire de données de mesure (3) pour stocker les données de mesure entrées ;
une mémoire de données de référence (4) pour stocker des données de référence sur la vision chromatique ; et
un moyen d'évaluation quantitative (5) pour évaluer quantitativement la lentille de la personne examinée en comparant les données de mesure et les données de référence,
dans lequel les données de mesure et les données de référence se rapportent toutes deux à des données de mesure de la vision chromatique pour une ou plusieurs couleurs spécifiques mesurées sur la base de deux images de test ou plus affichées sur un moniteur couleur, dont l'une est une image montrant un stimulus, et
dans lequel la mémoire de données de référence (4) stocke des données en tant que données de référence, les données étant des données de mesure de la vision chromatique d'un grand groupe de personnes ayant une lentille artificielle insérée dans l'œil et les données étant classées par types de lentilles artificielles.

2. Dispositif d'évaluation de lentille (1) selon la revendication 1, dans lequel la lentille est une lentille artificielle insérée dans un œil de la personne examinée à la suite d'une opération ou une lentille originale qui n'est pas la lentille artificielle.

3. Dispositif d'évaluation de lentille (1) selon la revendication 1 ou 2, dans lequel la mémoire de données de référence (4) stocke en outre des données de mesure de la vision chromatique d'un grand groupe de personnes qui n'installent pas de lentille artificielle en tant que données de référence.

4. Dispositif d'évaluation de lentille (1) selon la revendication 1, dans lequel la mémoire de données de référence (4) stocke les données de mesure de la vision chromatique, classées par âges.

5. Dispositif d'évaluation de lentille (1) selon la revendication 1 ou 4, dans lequel la mémoire de données de référence (4) stocke les données classées par types de lentilles artificielles, classées en outre par temps écoulé après l'opération.

6. Dispositif d'évaluation de lentille (1) selon l'une quelconque des revendications 1 à 5, comprenant en outre :
un moyen (6) d'entrée d'informations relatives à la personne examinée permettant d'entrer au moins une donnée relative aux types de lentilles des personnes examinées, à l'âge des personnes examinées et au temps écoulé après l'opération en tant qu'informations relatives à la personne examinée ;
une mémoire d'informations relatives à la personne examinée (7) pour stocker les informations entrées relatives à la personne examinée ;
un désignateur de mode d'évaluation (8) pour désigner avec quelles informations sur la personne examinée parmi les informations sur la personne examinée stockées dans la mémoire d'informations relatives à la personne examinée (7) et avec quelles données de référence parmi les données de référence stockées dans la mémoire de données de référence (4) l'évaluation quantitative doit être effectuée.

7. Dispositif d'évaluation de lentille (1) selon la revendication 6, dans lequel le moyen d'évaluation quantitative (5) compare les données de mesure sur deux couleurs différentes en fonction du type de lentille, de l'âge de la personne examinée ou du temps écoulé après l'opération, désignés par le désignateur de mode d'évaluation (8).

8. Programme de commande permettant de commander un ordinateur pour qu'il fonctionne comme suit :
un moyen d'entrée de données de mesure (2) pour entrer des données de mesure de la vision chromatique d'une personne examinée;
une mémoire de données de mesure (3) pour stocker les données de mesure entrées ;
une mémoire de données de référence (4) pour stocker des données de référence sur la vision chromatique ; et
un moyen d'évaluation quantitative (5) pour évaluer quantitativement une lentille de la personne examinée en comparant les données de mesure et les données de référence,
dans lequel les données de mesure et les données de référence se rapportent toutes deux à des données de mesure de la vision chromatique pour une ou plusieurs couleurs spécifiques mesurées sur la base de deux images de test ou plus affichées sur un moniteur couleur, dont l'une est une image montrant un stimulus, et
dans lequel la mémoire de données de référence (4) stocke des données en tant que données de référence, les données étant des données de mesure de la vision chromatique d'un grand groupe de personnes ayant une lentille artificielle insérée dans l'œil et les données étant classées par types de lentilles artificielles.

9. Programme de commande selon la revendication 8, dans lequel la mémoire de données de référence (4) stocke en outre des données de mesure de la vision chromatique d'un grand groupe de personnes qui n'installent pas de lentille artificielle en tant que données de référence.

10. Programme de commande selon la revendication 8, dans lequel la mémoire de données de référence (4) stocke les données de mesure de la vision chromatique, classées par âges.

11. Programme de commande selon la revendication 8 ou 10, dans lequel la mémoire de données de référence (4) stocke les données classées par types de lentilles artificielles, classées en outre par temps écoulé après l'opération.

12. Programme de commande selon l'une des revendications 8 à 11, permettant à l'ordinateur de fonctionner comme suit :
un moyen (6) d'entrée d'informations relatives à la personne examinée permettant d'entrer au moins une donnée relative aux types de lentilles des personnes examinées, à l'âge des personnes examinées et au temps écoulé après l'opération en tant qu'informations relatives à la personne examinée ;
une mémoire d'informations relatives à la personne examinée (7) pour stocker les informations entrées sur la personne examinée ;
un désignateur de mode d'évaluation (8) pour indiquer avec quelles informations sur la personne examinée parmi les informations sur la personne examinée stockées dans la mémoire d'informations relatives à la personne examinée (7) et avec quelles données de référence parmi les données de référence stockées dans la mémoire de données de référence (4) l'évaluation quantitative doit être effectuée.

13. Programme de commande selon la revendication 12, dans lequel le moyen d'évaluation quantitative (5) compare les données de mesure sur deux couleurs différentes en fonction du type de lentille, de l'âge de la personne examinée ou du temps écoulé après l'opération désignés par l'indicateur de mode d'évaluation (8).

14. Procédé de commande d'un dispositif d'évaluation de lentille (1) pour évaluer quantitativement la lentille d'une personne examinée, comprenant :
une étape d'entrée de données de mesure sur la vision chromatique de la personne examinée par le moyen d'entrée de données de mesure (2) ;
une étape de stockage de données de mesure entrées par l'intermédiaire d'une mémoire de données de mesure (3) ;
une étape de stockage de données de référence sur la vision chromatique par l'intermédiaire d'une mémoire de données de référence (4) ; et
une étape d'évaluation quantitative de la lentille de la personne examinée par un moyen d'évaluation quantitative (5) en comparant les données de mesure et les données de référence,
dans lequel les données de mesure et les données de référence se rapportent toutes deux à des données de mesure de la vision chromatique pour une ou plusieurs couleurs spécifiques mesurées sur la base de deux images de test ou plus affichées sur un moniteur couleur, dont l'une est une image montrant un stimulus, et
dans lequel la mémoire de données de référence (4) stocke des données en tant que données de référence, les données étant des données de mesure de la vision chromatique d'un grand groupe de personnes ayant une lentille artificielle insérée dans l'œil et les données étant classées par types de lentilles artificielles.

15. Procédé de commande du dispositif d'évaluation de lentille (1) selon la revendication 14, dans lequel la mémoire de données de référence (4) stocke en outre des données de mesure de la vision chromatique d'un grand groupe de personnes qui n'installent pas de lentille artificielle en tant que données de référence.

16. Procédé de commande du dispositif d'évaluation de lentille (1) selon la revendication 14, dans lequel la mémoire de données de référence (4) stocke les données de mesure de la vision chromatique, classées par âges.

17. Procédé de commande du dispositif d'évaluation des lentille (1) selon la revendication 14 ou 16, dans lequel la mémoire de données de référence (4) stocke les données classées par types de lentilles artificielles, classées en outre par temps écoulé après l'opération.

18. Procédé de commande du dispositif d'évaluation de lentille (1) selon l'une quelconque des revendications 14 à 17, comprenant en outre :
une étape consistant à entrer au moins une donnée relative aux types de lentilles des personnes examinées, à l'âge des personnes examinées et au temps écoulé après l'opération en tant qu'informations sur la personne examinée par l'intermédiaire du moyen (6) d'entrée d'informations relatives à la personne examinée ;
une étape de stockage d'informations entrées sur la personne examinée dans la mémoire d'informations relatives à la personne examinée (7) ; et
une étape consistant à désigner avec quelles informations sur la personne examinée parmi les informations sur la personne examinée stockées dans la mémoire d'informations relatives à la personne examinée (7) et avec quelles données de référence parmi les données de référence stockées dans la mémoire de données de référence (4) l'évaluation quantitative doit être effectuée par le biais du désignateur de mode d'évaluation (8).

19. Procédé de commande du dispositif d'évaluation de lentille (1) selon la revendication 18, dans lequel le moyen d'évaluation quantitative (5) compare les données de mesure sur deux couleurs différentes en fonction des types de lentilles, de l'âge des personnes examinées ou du temps écoulé après l'opération désignés par le désignateur de mode d'évaluation (8).
